# EUROPEAN PATENT SPECIFICATION

(11) **EP 3 240 529 B1**
(45) Date of publication and mention of the grant of the patent: **02.02.2022**
(21) Application number: 15817912.7
(22) Date of filing: 30.12.2015
(51) Int. Cl.: A61K 9/19, A61K 9/00, A61K 47/12, A61K 47/26, A61K 31/4164, A61P 31/10

(54) **STABLE PHARMACEUTICAL COMPOSITIONS COMPRISING MICAFUNGIN**
STABILE PHARMAZEUTISCHE ZUSAMMENSETZUNGEN MIT MICAFUNGIN
COMPOSITIONS PHARMACEUTIQUES STABLES COMPRENANT DE LA MICAFUNGINE

(30) Priority: 31.12.2014 EP 14200697
(43) Date of publication of application: 08.11.2017
(73) Proprietor: Galenicum Health S.L.U., 08950 Esplugues de Llobregat (ES)
(72) Inventor: ARROYO HIDALGO, Sergio, 08005 Barcelona (ES)
(74) Representative: Torrejón-Nieto, Javier
(86) International application number: PCT/EP2015/081384
(87) International publication number: WO 2016/107890

(56) References cited:
- WO-A1-01/02002
- US-A1- 2013 331 312
- JAMES A. SEARLES ET AL: "The ice nucleation temperature determines the primary drying rate of lyophilization for samples frozen on a temperature-controlled shelf", JOURNAL OF PHARMACEUTICAL SCIENCES, vol. 90, no. 7, 1 July 2001 (2001-07-01), pages 860-871, XP55257137, WASHINGTON, US ISSN: 0022-3549, DOI: 10.1002/jps.1039

## Description

The present invention relates to a stable pharmaceutical composition comprising micafungin, to a process for the manufacture of said pharmaceutical composition as well as to its use in the treatment of fungal infections and/or conditions arising from said infections.

### STATE OF THE ART

Invasive fungal infections (IFIs) are a frequent cause of morbidity and mortality in high-risk patients, such as immunocompromised patients. The number of such patients has increased in recent years due to increases in the use of intensive cancer chemotherapy as well as immunosuppressive regimens for autoimmune disease, the occurrence of solid organ and bone marrow transplantation, and the incidence of individuals with diseases of the immune system such as AIDS.

*Candida* is currently the predominant fungal pathogen in these patient populations. However, invasive aspergillosis has been increasing in incidence and *Aspergillus* is a significant fungal pathogen in bone marrow transplant recipients. Organisms belonging to both genera are associated with significant morbidity and a high mortality. Current therapies are not sufficient and treatment alternatives for this indication are urgently needed.

Micafungin (FK463), manufactured by Astellas Pharma Co., Ltd., is a water-soluble semisynthetic compound belonging to the new class of antifungal agents, the echinocandin lipopeptides. It is represented by the formula (I):

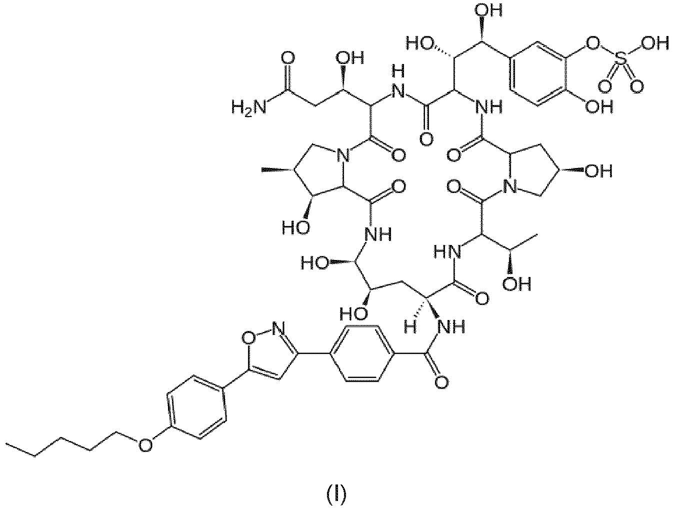

Micafungin is synthesised through the chemical modification of a fermentation product from Coleophoma empetri F-11899. It acts by selectively inhibiting 1,3-beta-D-glucan synthase, which is required for fungal cell wall synthesis. Micafungin was firstly marketed in Japan in 2002, and approved by FDA and marketed in US in May 2005. Clinical test has demonstrated that micafungin is very efficient for treating *Candida* and *Aspergillus*, and can be used as first-line medicine for treating diseases caused by *Candida* infections.

Echinocandins are inhibitors of fungal cell wall biosynthesis. Echinocandins exert their effect by disrupting the synthesis of 1,3-beta-D-glucan, an integral component of the fungal cell wall. Their mechanism of action differs from those of established classes of widely used systemic antifungal agents, which affect cell membrane sterols, either by direct interaction with them (polyenes, represented by amphotericin B) or through inhibition of sterol synthesis (e.g. azoles, including fluconazole, itraconazole and voriconazole), or interfere with nucleic acid metabolism (flucytosine). Echinocandins, including micafungin, are expected to show no cross-resistance to other antifungal agents.

Parenteral (ip) formulations of pharmaceutical drugs may be administered to patients via intramuscular (im), intravenous (iv) or subcutaneous methodology. The formulation that is developed for a particular drug is dependent on a variety of issues. If freeze-dried, the formulation should be capable of forming a well-formed cake and readily reconstitutable (usually in less than one minute). Finally, the formulation should have an acceptable appearance and be prepared from generally accepted, safe excipients.

Stability is an important consideration when designing a formulation. For practical reasons, it must be possible to store the formulation for at least two years. Therefore, it is often desirable to freeze dry the formulation to achieve better shelf-life and storage at room temperature.

The instability and poor water solubility (<0.1 mg/ml) of the echinocandin compounds make them particularly difficult to formulate. Most of the formulations tested to date have a shelf life of less than one year.

Micafungin is sensitive to high temperature, high humidity and exposure to light. This entails a further challenge in formulating an ip formulation containing said echinocandin compound. Therefore, there is a need for a formulation that improves the stability of the compound and the salts thereof.

EP1107777B1 discloses a stable pharmaceutical composition of micafungin in lyophilized form comprising lactose or maltose or sucrose as stabilizing agent. WO2012103801 discloses a pharmaceutical composition comprising micafungin and trehalose as stabilizing agent. WO2014173205 relates to a micafungin composition comprising a polysaccharide or disaccharide and which optionally may contain an appropriate amount of pH adjuster. However, none of these pharmaceutical compositions are ideal in stability.

Mycamine^{®} is used for treatment of invasive candidiasis. Each vial of Mycamine^{®} contains micafungin sodium as active substance and is presented as powder for solution for infusion. Before administration to a patient, the lyophilized product is reconstituted by adding a diluent and the desired amount of the diluted mixture is transferred to an infusion bag to be administered to the patient in need thereof.

Therefore, there is a need for stable pharmaceutical compositions comprising micafungin with low formation of impurities during storage. Moreover, it would be desirable to provide compositions having a good stability and a long shelf-life and which may be manufactured by a simple and fast method.

### DESCRIPTION OF THE INVENTION

The present invention provides a stable pharmaceutical composition comprising micafungin or a pharmaceutically acceptable salt thereof. It has been found that having a water content below 2.0% can stabilize micafungin pharmaceutical compositions. The inventors have surprisingly found that an antifungal composition according to the present invention comprising micafungin or a pharmaceutically acceptable salt thereof, wherein the water content of the lyophilised cake or powder is below 2.0 % by weight of the total amount of the lyophilised cake or powder, result in a pharmaceutical composition with low formation of degradation products during storage.

Micafungin is easily degraded by oxidation, acid, base, water and photolytic degradation, so it is necessary to provide compositions comprising said active agent which are stable. The present invention provides a highly stable composition both in lyophilised form and as a solution after reconstitution of the lyophilised cake or powder. Advantageously, lyophilised preparations are stable, can be stored and are easily reconstituted. Moreover, lyophilised preparations may be kept sterile and essentially free of insoluble matter.

The present invention provides a process for preparing a pharmaceutical composition comprising micafungin or a pharmaceutically acceptable salt thereof in the form of a lyophilised cake or powder as defined in the claims, wherein the water content of the lyophilised cake or powder is below 2.0 % by weight of the total amount of the lyophilised cake or powder.

The term "pharmaceutically acceptable" indicates that the substance or composition must be compatible chemically and/or toxicologically, with the other ingredients comprising a pharmaceutical composition, the mammal being treated therewith, and/or the route of administration of the composition.

In a preferred embodiment the water content of the lyophilised cake or powder is between 0.1 and 1.9 % by weight of the total amount of the lyophilised cake or powder, preferably is between 0.5 and 1.5 % by weight of the total amount of the lyophilised cake or powder.

The term "active ingredient" or "active agent" refers to a therapeutically active compound, as well as any prodrugs thereof and pharmaceutically acceptable salts, hydrates and solvates of the compound and the prodrugs.

The determination of water content is measured by the method described in European Pharmacopoeia 7.0th edition 2.5.12. (Water: Semi-micro determination). The European Pharmacopoeia (Ph. Eur.) specifies Karl Fischer volumetric titration to measure the water content of many solvents, chemicals and other substances.

The determination of water content is measured by the method described in European Pharmacopoeia 7.0th edition 2.5.32. (Water: Micro Determination). The Karl Fischer coulometric titration is restricted to the quantitative determination of small amounts of water.

Preservation of the active ingredient is possible because the greatly reduced water content inhibits the action of microorganisms and enzymes that would normally spoil or degrade said active ingredient.

The pharmaceutical composition prepared by the process according to the invention comprises at least one bulking agent in amount between 30 and 85 % by weight in respect of the total amount of the pharmaceutical composition. Preferably, it comprises between 40 and 70 % by weight of the bulking agent or bulking agents in respect of the total amount of the pharmaceutical composition. The bulking agent is selected from the group consisting of a saccharide such as glucose, mannose, galactose, ribose, fructose, trehalose, sucrose, lactose, raffinose, mannitol, maltose, inositol, or sorbitol; an oligomer such as cyclodextrin; a polymer such as glycogen, cellulose, starch, dextran, chitosan, hyaluronate, or polyvinylpyrrolidone; a protein such as gelatin or serum albumin; and a mixture thereof. Preferably, the bulking agent is selected from lactose, galactose, mannitol, trehalose and a mixture thereof. More preferably, the bulking agent is lactose. Even more preferably, the bulking agent is lactose monohydrate.

The term "bulking agent" refers to a pharmaceutically acceptable excipient which provides bulk to the formulation such that when unit dosage amounts of the solution are lyophilised in containers, such as sterile vials, the freeze-dried residue will be clearly discernible. Acceptable bulking agents include, but are not limited to, carbohydrates such as simple sugars such as dextrose, ribose, fructose and the like, alcohol sugars such as mannitol, inositol and sorbitol, disaccharides including sucrose and lactose, naturally occurring polymers such as starch, dextrans, chitosan, hyaluronate, proteins (e.g., gelatin and serum albumin) and glycogen, and synthetic monomers and polymers. Bulking agents for use in the present invention preferably also act as osmolytes (i.e., aid in making the liquid form of the formulation isotonic with normal human serum). The bulking agent may act as a stabilizing agent. The term "stabilizing agent" refers to a pharmaceutically acceptable excipient that enhances the chemical or physical stability of the active ingredient in the formulation. Suitable stabilizing agents include polyols (e. g., polyethylene and propylene glycols, and carbohydrates such as sucrose, trehalose, fructose, lactose and mannitol), amino acids and surfactants such as polysorbates and bile salts.

The pharmaceutical composition prepared by the process according to the invention further comprises a pH modifying agent or a mixture of pH modifying agents. Preferably, the pH modifying agent is an acid or a buffer. Preferably, the acid is citric acid, preferably anhydrous citric acid. The term "pH modifying agent" as used herein refers to an excipient capable of modifying the pH of the composition. The pH of the composition is that of the solution comprising micafungin or a pharmaceutically acceptable salt thereof before lyophilisation or after reconstitution of the lyophilised cake or powder.

In a preferred embodiment the micafungin is in amorphous form.

In a preferred embodiment the pharmaceutical composition comprises between 30 and 120 mg of micafungin as free base by unit dose. Preferably, the pharmaceutical composition comprises about 50 mg of micafungin as free base by unit dose or about 100 mg of micafungin as free base by unit dose. As used herein, the term "unit dose" or "unit dosage" refers to a physically discrete unit that contains a predetermined quantity of active ingredient calculated to produce a desired therapeutic effect. The unit dose or unit dosage may be in the form of a vial, tablet, capsule, sachet, etc. referred to herein as a "unit dosage form".

The lyophilized cake prepared by the process according to the invention is suitable for reconstitution to form a liquid composition for parenteral, preferably intravenous, administration.

The compositions of the present invention result in low formation of total impurities during storage compared with lyophilized formulations with a higher content of water known in the art. The term "total impurities" as used herein means the total amount of impurities commonly present in a pharmaceutically acceptable micafungin product or a pharmaceutically active micafungin salt prepared according to methods for preparing micafungin or a salt thereof well known to the skilled person in the art. The total amount of impurities present may be measured by HPLC-analysis. The change in the total amount of impurities during storage may be presented as the sum of the area percentage of the total amount of impurities in a formulation to be analysed. The person skilled in the art is familiar with various applicable HPLC devices and methods for measuring the formation of impurities during storage.

The pharmaceutical compositions of the invention are stable. The term "stable" as used herein refers to a pharmaceutical composition comprising micafungin wherein the total content of impurities originating from the decomposition of micafungin does not exceed 5 % area, preferably 3 % area and more preferably 2 % area determined by liquid chromatography (HPLC) at 275 nm if such a composition is stored for 6 months at 5 °C. Further, the term "stable" as used herein for lyophilized forms, refers to a pharmaceutical composition which when reconstituted in a sterile liquid vehicle, the reconstituted solution is clear without precipitation of the water insoluble drug for at least 2 hours after addition of the sterile liquid vehicle.

Once lyophilized, the pharmaceutical compositions as herein disclosed are packaged (e.g. the vials are stoppered) and ready for storing and/or shipping to end users. For use, the lyophilized cake or powder is reconstituted by adding a suitable reconstitution solution. Then, the reconstituted composition is further diluted in a bag for intravenous administration.

Suitable sterile solutions for reconstitution of the lyophilizate form of the pharmaceutical compositions as herein disclosed are distilled and/or sterile water for injection, bacteriostatic water for injection optionally comprising methylparabene and/or propylparabene and/or saline, 5 % glucose solution, 5 % or 10 % dextrose solution. Preferably, saline is 0.9 % sodium chloride, or 0.45 % or 0.225 % solution of sodium chloride. Also, a solution of 0.45 % sodium chloride and 2.5% dextrose can be used. Other suitable solutions can be 6 % dextran in 5 % dextrose or 6 to 10 % hydroxyethyl starch solutions in sterile water for injection.

The lyophilized cake prepared by the process according to the invention is reconstituted in a sterile aqueous solution, preferably the sterile aqueous solution is distilled and/or sterile water for injection, bacteriostatic water for injection optionally comprising saline, 5 % glucose solution, 5 % dextrose solution, Hartmann's solution, Ringer's solution and/or Ringer's lactate solution, more preferably the sterile aqueous solution is saline or 5 % glucose solution.

In a preferred embodiment the pH of the reconstituted pharmaceutical composition is between 3.0 and 8.0. Preferably, the pH of the reconstituted pharmaceutical composition is between 3.5 and 7.0. More preferably, the pH of the reconstituted pharmaceutical composition is between 4.5 and 6.0.

The determination of pH is measured by the method described in the European Pharmacopoeia 7.0th edition 2.2.3. (Potentiometric determination of pH). The pH is a number which represents conventionally the hydrogen ion concentration of an aqueous solution. The potentiometric determination of pH is made by measuring the potential difference between 2 appropriate electrodes immersed in the solution to be examined. A Crison pH meter GLP21 was used. This instrument uses a pH electrode sensor A.T.C. type Pt 1000, measures electric potential differences with a high entry impedance and high resolution. The instrument was calibrated before the first measurement and after each 15 measurements with 2 reference solutions having pH values of 4.00 and 7.02.

The present invention provides a process for the manufacture of a pharmaceutical composition comprising micafungin, comprising at least the step of dissolving micafungin in a water solution in a reactor made of a material other than glass, the material being defined in the claims.

The inventors have surprisingly found that the process for the manufacture of a pharmaceutical composition comprising micafungin avoiding the use of reactor made of glass is more consistent and robust in terms of reproducibility and minimizing losses. In the process of the present invention, drug substance retention in the glass material is avoided and therefore the loss of said drug substance is minimized.

In a preferred embodiment of the process of the present invention, the reactor material is selected from the group consisting of stainless steel, carbon steel, teflon, high alloy steel and high alloys such as hastelloy, tantalum, inconel, monel, titanium, nickel and cupronickel, preferably the reactor is a stainless steel reactor.

The term "reactor" as used herein refers to a vessel in the chemical processing industries that is used for a variety of process operations, including: product mixing, chemical reactions, batch distillation, liquid extraction, polymerization, solids dissolution, and other forms of reaction, mixing, or catalyst functions. A reactor consists of a tank or body with an agitator and heating or cooling system. Vessels are usually fabricated with stainless steel, carbon steel or high alloy steel depending on the corrosiveness of the chemicals or products being used.

In a preferred embodiment the process comprises at least the following steps:
i) preparing an aqueous solution of at least one pharmaceutically acceptable excipient in water in a reactor made of stainless steel, carbon steel, high alloy steel and high alloys such as hastelloy, tantalum, inconel, monel, titanium, nickel and cupronickel, preferably the pharmaceutically acceptable excipient is lactose, more preferably lactose monohydrate;
ii) optionally heating the solution of step (i) to a temperature between 35°C and 45°C to dissolve the pharmaceutically acceptable excipient;
iii) when appropriate, cooling the solution of step (i) or (ii) to a temperature between 5°C and 15°C to form a cooled solution;
iv) adding under stirrer agitation a pharmaceutically acceptable amount of micafungin to the solution obtained in step (i), (ii) or (iii);
v) adjusting the pH of the solution obtained in step (iv) to a pH between 3.5 and 7.0, preferably to between 4.6 and 6.0 using a pharmaceutically acceptable amount of a pH modifying agent;
vi) optionally filtering the solution obtained in step (iv) or (v);
vii)freezing the solution obtained in step (iv), (v) or (vi), preferably this step is performed in a vial, more preferably the vial is sealed after freeze-drying under an inert atmosphere, preferably under nitrogen or argon atmosphere; and
viii) freeze-drying the frozen solution obtained in step (vii), preferably this step is performed in a vial, more preferably the vial is sealed after freeze-drying under an inert atmosphere, preferably under nitrogen or argon atmosphere.

In a preferred embodiment of the process the pharmaceutically acceptable excipient in step (i) is lactose, more preferably lactose monohydrate and the pH modifying agent used in step (v) is selected from the group consisting of citric acid, NaOH and mixtures thereof. Preferably, the pH is adjusted to between 4.5 and 6.0 in step (v). More preferably, nitrogen gas is bubbled in the solution. Even more preferably, steps (vii) and/or (viii) are performed in a vial and the vial is sealed after freeze-drying under an inert atmosphere, preferably under nitrogen or argon atmosphere.

The process according to the invention involves a dissolution and mixing of the ingredients, filtration of the obtained mixture, and transfer of the solution to suitable vials. The so obtained solution is lyophilized to obtain a lyophilized cake. Lyophilization, also called freeze-drying, refers to a drying process by freezing a material dissolved in water below 0 °C and then reducing the surrounding pressure using a vacuum pump to allow the frozen water in the material to sublimate directly from the solid phase to the gas phase. The composition is preferably lyophilized in pharmaceutically acceptable vials according to the method of the present invention.

Another aspect of the process of the present invention is the duration of the freeze drying that is less than 50 hours long, preferably between 10 and 45 hours long, and more preferably between 25 and 40 hours long from the initial step of freezing the solution until the end of the secondary drying.

In a preferred embodiment of the process, the freeze drying comprises the following steps:
i) freezing the solution at a temperature below -20 °C and maintaining said temperature for at least 1 hour;
ii) primary drying the frozen solution of step (i) under vacuum and at a temperature between -10 and 10 °C, and maintaining said conditions for at least 10 hours; and
iii) secondary drying the primary dried frozen solution of step (ii) under vacuum and at a temperature between 20 and 45 °C, and maintaining said conditions for at least 5 hours.

The lyophilisation (freeze-drying) process as disclosed herein comprises at least three steps: freezing, primary drying, and secondary drying. The lyophilisation process as herein disclosed provides a good quality of the resulting cake in the minimum time. This process maximizes the rate of heat transfer to each vial without causing cake collapse and avoiding slower reconstitution times. The freeze drying cycle is efficient and robust without compromising the pharmaceutical quality of the product and neither other parameters such as stability and water content.

The term "primary drying" refers to heating the material at low temperature and low pressure to cause frozen "free water" to sublimate directly to vapour. The term "secondary drying", also known as desorption drying, refers to further heating the material, so that the bound unfrozen water absorbs the heat of desorption and becomes free water, which then absorbs the heat of evaporation and becomes vapour, finally escaping from the material.

In a preferred embodiment, the temperature in step (i) is between -55 and -35 °C, preferably between -50 and -40 °C, maintained at least 2 hours, preferably between 2 and 4 hours. In a more preferred embodiment, the temperature in step (ii) is between -5 and 10 °C, maintained at least 12 hours, preferably between 15 and 25 hours. In a more preferred embodiment, the temperature in step (iii) is between 25 and 40 °C, maintained at least 7 hours, preferably between 8 and 16 hours. In a more preferred embodiment, the temperature in step (i) is between -55 and -35 °C, preferably between -50 and -40 °C, maintained at least 2 hours, preferably between 2 and 4 hours; and the temperature in step (ii) is between -5 and 10 °C, maintained at least 12 hours, preferably between 15 and 25 hours; and the temperature in step (iii) is between 25 and 40 °C, maintained at least 7 hours, preferably between 8 and 16 hours.

In a preferred embodiment, the vacuum in step (ii) ranges from 0.01 to 1 mbar, preferably from 0.05 to 0.5 mbar, more preferably from 0.1 to 0.2 mbar. In a preferred embodiment, the vacuum in step (iii) ranges from 0.005 to 0.1 mbar, preferably from 0.01 to 0.08 mbar, more preferably from 0.02 to 0.06 mbar. Unit conversion: 1 mbar = 100 Pa.

Further there is described a pharmaceutical batch comprising 1,200 units of the pharmaceutical composition prepared by the process according to the invention. Preferably, the pharmaceutical batch comprises at least 6,000 units.

Another aspect relates to the pharmaceutical composition prepared by the process according to the invention, or the pharmaceutical batch prepared by the process according to the invention, for use in the treatment of a fungal infection in a mammal.

The lyophilized composition is then appropriately sealed and stored (e.g., in stoppered vials) for later use. In a further embodiment, the vial as herein disclosed has a volume between 3 and 12 ml, preferably between 4 and 11 ml, more preferably it is a 10 ml vial. More preferably, the vial is capped with a rubber stopper and a seal.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP. A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active ingredient in the vials of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

There is further described a glass vial comprising the pharmaceutical composition prepared according to the process of the invention.

There is further described a cardboard box with a patient information leaflet comprising at least one unit of the pharmaceutical composition prepared according to the process of the present invention.

There is further described a method for preparing a pharmaceutical dossier to obtain the marketing authorization of pharmaceutical composition prepared according to the process of the present invention comprising the following steps:
i) manufacturing at least one pharmaceutical batch;
ii) performing stability tests of the batches of step (i);
iii) compiling the results obtained in steps (i) to (iii); and
iv) providing the compiled results of step (iii) in a data carrier.

There is provided a data carrier comprising the compiled results of a pharmaceutical dossier obtained by the method as described in the previous paragraph.

The term "pharmaceutical dossier" to obtain the marketing authorization refers to a dossier with data proving that the drug has quality, efficacy and safety properties suitable for the intended use, additional administrative documents, samples of finished product or related substances and reagents necessary to perform analyzes of finished product as described in that dossier.

The data carrier is selected from the group consisting of a digital data carrier such as CD, DVD, USB, hard drive; and paper.

The term "data carrier" refers to a device for recording (storing) information (data). Recording can be done using virtually any form of energy. A storage device may hold information, process information, or both. Most often the term is used with computers. Data carrier can permanently hold data, like files.

Unless otherwise indicated, all the analysis methods are carried out according to the European Pharmacopoeia 7th edition.

All percentages, parts and ratios herein disclosed are by weight unless specifically noted otherwise. As used herein, the term "about" refers preferably to a range that is +-10 %, preferably +-5 %, or more preferably the value with which the term is associated.

### EXAMPLES

The following examples illustrate various embodiments of the invention and are not intended to limit the invention in any way:

### Example 1: preparation of stable micafungin compositions.

Lactose monohydrate was dissolved in 80 % of the total quantity of water for injection under heating to 40 °C. The solution was stirred for about 10 minutes until complete dissolution in a stainless steel reactor. The solution was cooled below 15 °C and micafungin was added while stirring in a uniform speed to avoid foam formation. 1.025 ml of 0.76 % by weight of anhydrous citric acid of was added to the solution. The pH of the solution was then adjusted to between 4.5 and 6.0 with 0.384 % by weight of NaOH, and the volume of the solution was adjusted by adding the required amount of water for injection. The solution was sterile-filtered through a 0.22 µm pore size syringe filter. The solution was thereafter transferred to 10 ml lyophilisation vials and pre-stopped with sterile butyl rubber stoppers and a flip-off cap. The solution was then subjected to lyophilisation. The vial was wrapped with an UV-protective film.

**Table 1. Compositions of examples 1a and 1b.**

| | Ex. 1a | Ex. 1b |
|---|---|---|
| Unit dose | 50 mg/vial | 100 mg/vial |
| Micafungin sodium (mg/vial) | 50.86 | 101.73 |
| Lactose monohydrate (mg/vial) | 210.5 | 210.5 |
| Anhydrous citric acid (mg/vial) | 0.19 | 0.19 |
| NaOH 0.384% ⁽¹⁾ | q.s. to pH 4.5-6.0 | q.s. to pH 4.5-6.0 |
| Purified Water ⁽²⁾ | q.s. to 4.37 ml | q.s. to 4.37 ml |

| | | |
|---|---|---|
| (1) "q.s. to pH" means the addition of alkali in a quantity sufficient to bring the solution to a desired pH (e.g., q.s. to pH 4.5-6.0 means the addition of alkali to bring the solution to a pH of 4.5-6.0). (2) "q.s." means adding a quantity sufficient to achieve a certain state (e.g., volume, i.e., to bring a solution to a desired volume). * Vials were overfilled with 4 % to fill the vials with 4.36 ml of the filtered solution. | | |

The compositions of examples 1a and 1b were prepared at the same time and subjected to the same lyophilisation cycle in order to minimize external factors that could affect the final composition.

### Example 2: Lyophilization process

The compositions prepared in example 1a and 1b were subjected to lyophilization. Two batches of 350 vials/batch were lyophilized following the process described in the embodiments of the description of the present invention.

The freeze drying of the pharmaceutical compositions of the examples was performed at different temperatures, pressures and durations and it was found that good cakes with no collapse or cavities were obtained when the freeze drying took at least 16 hours and less than 50 hours, from the start of the freezing of the solution to the end of the secondary drying. Also, very good cakes were obtained when the solution was frozen at a temperature below -20 °C and when the primary drying was set at a temperature between -10 and 10 °C, and was maintained for at least 10 hours. Very good cakes were obtained when the secondary drying temperature was set between 20 and 40 °C, and maintained for at least 5 hours.

### Example 3: Stability Studies for the compositions disclosed in example 1.

The compositions prepared according to examples 1a and 1b are tested for stability at time zero after lyophilisation and also are stored in the lyophilised state at 40 °C and 75 % of relative humidity (RH) for 3 months. Prior to testing, the lyophilised material was dissolved in a pharmaceutically acceptable reconstitution solution. The so obtained solutions were then analysed by HPLC according to standard methods well known to the skilled person in the art.

### Example 4: Preparation of stable micafungin compositions.

Lactose monohydrate was dissolved in 80 % of the total quantity of water for injection under heating to 40 °C. The solution was stirred for about 10 minutes until complete dissolution in a reactor. The solution was cooled below 15 °C and micafungin was added while stirring in a uniform speed to avoid foam formation. 1.025 ml of 0.76 % by weight of anhydrous citric acid of was added to the solution. The pH of the solution was then adjusted to between 4.5 and 6.0 with 0.384 % by weight of NaOH, and the volume of the solution was adjusted by adding the required amount of water for injection. The solution was sterile-filtered through a 0.22 µm pore size syringe filter. The solution was thereafter transferred to 10 ml lyophilisation vials and pre-stopped with sterile butyl rubber stoppers and a flip-off cap. The solution was then subjected to lyophilisation as described in example 2. The vial was wrapped with an UV-protective film.

**Table 2. Compositions of examples 4a and 4b using reactors made of different materials.**

| | Ex. 4a | Ex. 4b |
|---|---|---|
| Unit dose | 100 mg/vial | 100 mg/vial |
| Micafungin sodium (mg/vial) | 101.73 | 101.73 |
| Lactose monohydrate (mg/vial) | 210.5 | 215.8 |
| Anhydrous citric acid (mg/vial) | 0.19 | 0.19 |
| NaOH 0.384% ⁽¹⁾ | q.s. to pH 4.5-6.0 | q.s. to pH 4.5-6.0 |
| Purified Water ⁽²⁾ | q.s. to 4.46 ml | q.s. to 4.30 ml |
| Reactor material | Stainless steel | Glass |

| | | |
|---|---|---|
| (1) "q.s. to pH" means the addition of alkali in a quantity sufficient to bring the solution to a desired pH (e.g., q.s. to pH 4.5-6.0 means the addition of alkali to bring the solution to a pH of 4.5-6.0). (2) "q.s." means adding a quantity sufficient to achieve a certain state (e.g., volume, i.e., to bring a solution to a desired volume). * Ex 4a: vials were overfilled with 6.3 % to fill the vials with 4.46 ml of the filtered solution. * Ex 4b: vials were overfilled with 2.5 % to fill the vials with 4.30 ml of the filtered solution. | | |

**Table 3. Assay (%) results of examples 4a and 4b.**

| | Assay (%) |
|---|---|
| Ex 4a (vial lyophilized 1 | 99.3 |
| Ex 4a (vial lyophilized 2) | 99.2 |
| Ex 4a (before lyophilization 1) | 99.9 |
| Ex 4a (before lyophilization 2) | 99.9 |
| Ex 4b (vial lyophilized 1) | 93.2 |
| Ex 4b (vial lyophilized 2) | 96.2 |
| Ex 4b (before lyophilization 1) | 94.6 |
| Ex 4b (before lyophilization 2) | 93.5 |

The compositions of examples 4a and 4b were prepared at the same time and subjected to the same lyophilisation cycle in order to minimize external factors that could affect the final composition. Assay (%) results in the pharmaceutical composition prepared using a stainless steel reactor are more consistent and robust in terms of reproducibility than the ones obtained in the pharmaceutical composition using a reactor of glass material. In the process using a reactor made of a material other than glass, drug substance retention in the glass material is avoided and therefore the loss of said drug substance is minimized.

### Example 5: Water content.

The amount of water of the pharmaceutical compositions as herein disclosed was measured by Karl Fisher titration.

**Table 4. Water content.**

| | Ex. 1a | Ex. 1b Ex. 4a Ex. 4b | | |
|---|---|---|---|---|
| Water content (%) | 0.9 | 1.1 | 1.1 | 1.2 |

## Claims

1. A process for preparing a pharmaceutical composition comprising micafungin or a pharmaceutically acceptable salt thereof in the form of a lyophilised cake or powder,
wherein the water content of the lyophilised cake or powder is below 2.0 % by weight, preferably between 0.1 and 1.9 % by weight, more preferably between 0.5 and 1.5 % by weight of the total amount of the lyophilised cake or powder, wherein the pharmaceutical composition comprises at least one bulking agent, wherein the pharmaceutical composition comprises between 30 and 85 % by weight, preferably between 40 and 70 % by weight of the bulking agent or bulking agents in respect of the total amount of the pharmaceutical composition wherein the process comprises, at least, the step of preparing a micafungin solution by dissolving micafungin in a water solution in a reactor made of stainless steel, carbon steel, high alloy steel and high alloys such as hastelloy, tantalum, inconel, monel, titanium, nickel and cupronickel, preferably the reactor is a stainless steel reactor.

2. The process according to the preceding claim, wherein the pharmaceutical composition comprises at least one bulking agent and the bulking agent is selected from the group consisting of a saccharide such as glucose, mannose, galactose, ribose, fructose, trehalose, sucrose, lactose, raffinose, mannitol, maltose, inositol, or sorbitol; an oligomer such as cyclodextrin; a polymer such as glycogen, cellulose, starch, dextran, chitosan, hyaluronate, or polyvinylpyrrolidone; a protein such as gelatin or serum albumin; and a mixture thereof, preferably the bulking agent is selected from lactose, galactose, mannitol, trehalose and a mixture thereof, more preferably the bulking agent is lactose, even more preferably the bulking agent is lactose monohydrate.

3. The process according to the preceding claims, wherein the pharmaceutical composition further comprises a pH modifying agent or a mixture of pH modifying agents, preferably the pH modifying agent is an acid ora buffer, more preferably the acid is citric acid, even more preferably anhydrous citric acid.

4. The process according to the preceding claims, wherein the micafungin is in amorphous form.

5. The process according to the preceding claims, wherein the pharmaceutical composition comprises between 30 and 120 mg of micafungin as free base by unit dose, preferably about 50 mg of micafungin as free base by unit dose or about 100 mg of micafungin as free base by unit dose.

6. The process according to the preceding claims, wherein the pharmaceutical composition is suitable for reconstitution to form a liquid composition for parenteral, preferably intravenous, administration.

7. The process according to the preceding claims, comprising at least the following steps:
i) preparing an aqueous solution of at least one pharmaceutically acceptable excipient in water in a reactor made of stainless steel, carbon steel, high alloy steel and high alloys such as hastelloy, tantalum, inconel, monel, titanium, nickel and cupronickel, preferably the pharmaceutically acceptable excipient is lactose, more preferably lactose monohydrate;
ii) optionally heating the solution of step (i) to a temperature between 35°C and 45 °C to dissolve the pharmaceutically acceptable excipient;
iii) when appropriate, cooling the solution of step (i) or (ii) to a temperature between 5°C and 15°C to form a cooled solution;
iv) adding under stirrer agitation a pharmaceutically acceptable amount of micafungin to the solution obtained in step (i), (ii) or (iii);
v) adjusting the pH of the solution obtained in step (iv) to a pH between 3.5 and 7.0, preferably to between 4.6 and 6.0 using a pharmaceutically acceptable amount of a pH modifying agent;
vi) optionally filtering the solution obtained in step (iv) or (v);
vii) freezing the solution obtained in step (iv), (v) or (vi), preferably this step is performed in a vial, more preferably the vial is sealed after freeze-drying under an inert atmosphere, preferably under nitrogen or argon atmosphere; and
viii) freeze drying the frozen solution obtained in step (vii), preferably this step is performed in a vial, more preferably the vial is sealed after freeze-drying under an inert atmosphere, preferably under nitrogen or argon atmosphere.

8. The process according to the previous claim wherein said freeze drying step comprises the steps of freezing the solution, primary drying and secondary drying, and wherein the freeze drying is less than 50 hours long, preferably between 10 and 45 hours long, and more preferably between 25 and 40 hours long from the initial step of freezing the solution until the end of the secondary drying.

9. The process according to the preceding claim, wherein the freeze drying comprises the following steps:
i) freezing the solution at a temperature below -20 °C and maintaining said temperature for at least 1 hour, preferably temperature is between -55 and -35 °C, more preferably between -50 and -40 °C, maintained at least 2 hours, more preferably between 2 and 4 hours;
ii) primary drying the frozen solution of step (i) under vacuum and at a temperature between -10 and 10°C, and maintaining said conditions for at least 10 hours, preferably is between -5 and 10 °C, maintained at least 12 hours, preferably between 15 and 25 hours; and
iii) secondary drying the primary dried frozen solution of step (ii) under vacuum and at a temperature between 20 and 45 °C, preferably temperature is between 25 and 40 °C, maintained at least 7 hours, more preferably between 8 and 16 hours, and maintaining said conditions for at least 5 hours.

10. The process according to the preceding claim, wherein the vacuum in step (ii) ranges from 1 Pa to 100 Pa (0.01 to 1 mbar), preferably 5 Pa to 50 Pa (0.05 to 0.5 mbar), more preferably from 10 Pa to 20 Pa (0.1 to 0.2 mbar) and/or the vacuum in step (iii) ranges from 0.5 Pa to 10 Pa (0.005 to 0.1 mbar), preferably from 1 Pa to 8 Pa (0.01 to 0.08 mbar), more preferably from 2 Pa to 6 Pa (0.02 to 0.06 mbar).

## Patentansprüche

1. Verfahren zum Herstellen einer pharmazeutischen Zusammensetzung, umfassend Micafungin oder ein pharmazeutisch verträgliches Salz davon in Form eines lyophilisierten Kuchens oder Pulvers,
wobei der Wassergehalt des lyophilisierten Kuchens oder Pulvers unter 2,0 Gew.-%, vorzugsweise zwischen 0,1 und 1,9 Gew.-%, stärker bevorzugt zwischen 0,5 und 1,5 Gew.-% der Gesamtmenge des lyophilisierten Kuchens oder Pulvers liegt, wobei die pharmazeutische Zusammensetzung mindestens einen Füllstoff umfasst, wobei die pharmazeutische Zusammensetzung zwischen 30 und 85 Gew.-%, vorzugsweise zwischen 40 und 70 Gew.-% des Füllstoffs oder der Füllstoffe in Bezug auf die Gesamtmenge der pharmazeutischen Zusammensetzung umfasst
wobei das Verfahren mindestens den Schritt des Herstellens einer Micafunginlösung durch Auflösen von Micafungin in einer Wasserlösung in einem Reaktor aus rostfreiem Stahl, Kohlenstoffstahl, hochlegiertem Stahl und hochlegierten Legierungen wie Hastelloy, Tantal, Inconel, Monel, Titan, Nickel und Kupfernickel umfasst, wobei der Reaktor vorzugsweise ein Edelstahlreaktor ist.

2. Verfahren nach dem vorstehenden Anspruch, wobei die pharmazeutische Zusammensetzung mindestens einen Füllstoff umfasst und der Füllstoff ausgewählt ist aus der Gruppe bestehend aus einem Saccharid wie Glucose, Mannose, Galactose, Ribose, Fructose, Trehalose, Saccharose, Lactose, Raffinose, Mannitol, Maltose, Inosit oder Sorbit; einem Oligomer wie Cyclodextrin; einem Polymer wie Glykogen, Cellulose, Stärke, Dextran, Chitosan, Hyaluronat oder Polyvinylpyrrolidon; einem Protein wie Gelatine oder Serumalbumin; und einer Mischung davon, wobei der Füllstoff vorzugsweise ausgewählt ist aus Lactose, Galactose, Mannitol, Trehalose und einer Mischung davon, wobei der Füllstoff stärker bevorzugt Lactose ist, der Füllstoff noch stärker bevorzugt Lactosemonohydrat ist.

3. Verfahren nach den vorstehenden Ansprüchen, wobei die pharmazeutische Zusammensetzung ferner ein pH-Modifizierungsmittel oder eine Mischung von pH-Modifizierungsmitteln umfasst, wobei das pH-Modifizierungsmittel vorzugsweise eine Säure oder ein Puffer ist, stärker bevorzugt die Säure Zitronensäure, noch stärker bevorzugt wasserfreie Zitronensäure ist.

4. Verfahren nach den vorstehenden Ansprüchen, wobei das Micafungin in amorpher Form vorliegt.

5. Verfahren nach den vorstehenden Ansprüchen, wobei die pharmazeutische Zusammensetzung zwischen 30 und 120 mg Micafungin als freie Base nach Einheitsdosis, vorzugsweise etwa 50 mg Micafungin als freie Base nach Einheitsdosis oder etwa 100 mg Micafungin als freie Base nach Einheitsdosis umfasst.

6. Verfahren nach den vorstehenden Ansprüchen, wobei die pharmazeutische Zusammensetzung zur Rekonstitution geeignet ist, um eine flüssige Zusammensetzung zur parenteralen, vorzugsweise intravenösen Verabreichung zu bilden.

7. Verfahren nach den vorstehenden Ansprüchen, umfassend mindestens die folgenden Schritte:
i) Herstellen einer wässrigen Lösung mindestens eines pharmazeutisch verträglichen Hilfsstoffs in Wasser in einem Reaktor aus rostfreiem Stahl, Kohlenstoffstahl, hochlegiertem Stahl und hochlegierten Legierungen wie Hastelloy, Tantal, Inconel, Monel, Titan, Nickel und Kupfernickel, wobei der pharmazeutisch verträgliche Hilfsstoff vorzugsweise Lactose, stärker bevorzugt Lactosemonohydrat ist;
ii) wahlweise Erwärmen der Lösung von Schritt (i) auf eine Temperatur zwischen 35 °C und 45 °C, um den pharmazeutisch verträglichen Hilfsstoff zu lösen;
iii) gegebenenfalls Abkühlen der Lösung von Schritt (i) oder (ii) auf eine Temperatur zwischen 5 °C und 15 °C, um eine abgekühlte Lösung zu bilden;
iv) Hinzufügen einer pharmazeutisch verträglichen Menge Micafungin zu der in Schritt (i), (ii) oder (iii) erhaltenen Lösung unter Rühren;
v) Einstellen des pH-Werts der in Schritt (iv) erhaltenen Lösung auf einen pH-Wert zwischen 3,5 und 7,0, vorzugsweise zwischen 4,6 und 6,0, unter Verwendung einer pharmazeutisch verträglichen Menge eines pH-Modifizierungsmittels;
vi) wahlweise Filtrieren der in Schritt (iv) oder (v) erhaltenen Lösung;
vii) Einfrieren der in Schritt (iv), (v) oder (vi) erhaltenen Lösung, wobei dieser Schritt vorzugsweise in einem Fläschchen durchgeführt, stärker bevorzugt das Fläschchen nach dem Gefriertrocknen unter einer inerten Atmosphäre, vorzugsweise unter Stickstoff- oder Argonatmosphäre, verschlossen wird; und
viii) Gefriertrocknen der in Schritt (vii) erhaltenen gefrorenen Lösung, wobei dieser Schritt vorzugsweise in einem Fläschchen durchgeführt, stärker bevorzugter das Fläschchen nach dem Gefriertrocknen unter einer inerten Atmosphäre, vorzugsweise unter Stickstoff- oder Argonatmosphäre, verschlossen wird.

8. Verfahren nach dem vorstehenden Anspruch, wobei der Gefriertrocknungsschritt die Schritte des Einfrierens der Lösung, des primären Trocknens und des sekundären Trocknens umfasst und wobei das Gefriertrocknen vom ersten Schritt des Einfrierens der Lösung bis zum Ende des sekundären Trocknens weniger als 50 Stunden lang ist, vorzugsweise zwischen 10 und 45 Stunden lang und stärker bevorzugt zwischen 25 und 40 Stunden lang ist.

9. Verfahren nach dem vorstehenden Anspruch, wobei das Gefriertrocknen die folgenden Schritte umfasst:
i) Einfrieren der Lösung bei einer Temperatur unter -20 °C und Aufrechterhalten der Temperatur für mindestens 1 Stunde, vorzugsweise zwischen -55 und -35 °C, stärker bevorzugt zwischen -50 und -40 °C, für mindestens 2 Stunden, stärker bevorzugt zwischen 2 und 4 Stunden;
ii) primäres Trocknen der gefrorenen Lösung von Schritt (i) unter Vakuum und bei einer Temperatur zwischen -10 und 10°C, und Aufrechterhalten der Bedingungen für mindestens 10 Stunden, wobei sie vorzugsweise zwischen -5 und 10°C liegt, aufrechterhalten für mindestens 12 Stunden, vorzugsweise zwischen 15 und 25 Stunden; und
iii) sekundäres Trocknen der primär getrockneten gefrorenen Lösung von Schritt (ii) unter Vakuum und bei einer Temperatur zwischen 20 und 45 °C, wobei die Temperatur vorzugsweise zwischen 25 und 40 °C liegt, aufrechterhalten für mindestens 7 Stunden, stärker bevorzugt zwischen 8 und 16 Stunden, und Aufrechterhalten der Bedingungen für mindestens 5 Stunden.

10. Verfahren nach dem vorstehenden Anspruch, wobei das Vakuum in Schritt (ii) im Bereich von 1 Pa bis 100 Pa (0.01 bis 1 mbar), vorzugsweise von 5 Pa bis 50 Pa (0.05 bis 0.5 mbar), stärker bevorzugt von 10 Pa bis 20 Pa (0.1 bis 0.2 mbar) liegt und/oder
das Vakuum in Schritt (iii) im Bereich von 0.5 Pa bis 10 Pa (0.005 bis 0.1 mbar), vorzugsweise von 1 Pa bis 8 Pa (0.01 bis 0.08 mbar), stärker bevorzugt 2 Pa bis 6 Pa (0.02 bis 0.06 mbar) liegt.

## Revendications

1. Procédé de préparation d'une composition pharmaceutique comprenant de la micafungine ou un sel pharmaceutiquement acceptable de celle-ci sous la forme d'un gâteau ou d'une poudre lyophilisée,
dans lequel la teneur en eau du gâteau ou de la poudre lyophilisée est inférieure à 2,0% en poids, de préférence entre 0,1 et 1,9% en poids, plus préférentiellement entre 0,5 et 1,5 % en poids de la quantité totale du gâteau ou de la poudre lyophilisée, dans lequel la composition pharmaceutique comprend au moins un agent gonflant, dans lequel la composition pharmaceutique comprend entre 30 et 85 % en poids, de préférence entre 40 et 70 % en poids de l'agent gonflant ou des agents gonflants par rapport à la quantité totale de la composition pharmaceutique
dans lequel le procédé comprend, au moins, l'étape de préparation d'une solution de micafungine par dissolution de micafungine dans une solution aqueuse dans un réacteur en acier inoxydable, en acier au carbone, en acier fortement allié et en alliages forts tels que l'hastelloy, le tantale, l'Inconel, le monel, le titane, le nickel et le cupronickel, de préférence, le réacteur est un réacteur en acier inoxydable.

2. Procédé selon la revendication précédente, dans lequel la composition pharmaceutique comprend au moins un agent gonflant et l'agent gonflant est choisi dans le groupe constitué d'un saccharide tel que le glucose, le mannose, le galactose, le ribose, le fructose, le tréhalose, le saccharose, le lactose, le raffinose, le mannitol, le maltose, l'inositol, ou le sorbitol ; un oligomère tel que la cyclodextrine; un polymère tel que le glycogène, la cellulose, l'amidon, le dextrane, le chitosane, l'hyaluronate ou le polyvinylpyrrolidone ; une protéine telle que la gélatine ou l'albumine sérique ; et un mélange de ces derniers, de préférence l'agent gonflant est choisi parmi le lactose, le galactose, le mannitol, le tréhalose et un mélange de ces derniers, plus préférablement, l'agent gonflant est le lactose, encore plus préférablement, l'agent gonflant est le lactose monohydraté.

3. Procédé selon les revendications précédentes, dans lequel la composition pharmaceutique comprend en outre un agent modifiant le pH ou un mélange d'agents modifiant le pH, de préférence, l'agent modifiant le pH est un acide ou un tampon, plus préférablement, l'acide est l'acide citrique, encore plus préférentiellement l'acide citrique anhydre.

4. Procédé selon les revendications précédentes, dans lequel la micafungine est sous forme amorphe.

5. Procédé selon les revendications précédentes, dans lequel la composition pharmaceutique comprend entre 30 et 120 mg de micafungine sous forme de base libre par dose unitaire, de préférence environ 50 mg de micafungine sous forme de base libre par dose unitaire ou environ 100 mg de micafungine sous forme de base libre par dose unitaire.

6. Procédé selon les revendications précédentes, dans lequel la composition pharmaceutique est appropriée pour une reconstitution pour former une composition liquide pour une administration parentérale, de préférence, intraveineuse.

7. Procédé selon les revendications précédentes, comprenant au moins les étapes suivantes :
i) préparation d'une solution aqueuse d'au moins un excipient pharmaceutiquement acceptable dans l'eau dans un réacteur en acier inoxydable, en acier au carbone, en acier fortement allié et en alliages forts tels que l'hastelloy, le tantale, l'Inconel, le monel, le titane, le nickel et le cupronickel, de préférence l'excipient pharmaceutiquement acceptable est le lactose, plus préférablement le lactose monohydraté ;
ii) facultativement, chauffage de la solution de l'étape (i) à une température entre 35 °C et 45 °C pour dissoudre l'excipient pharmaceutiquement acceptable ;
iii) le cas échéant, refroidissement de la solution de l'étape (i) ou (ii) à une température entre 5 °C et 15 °C pour former une solution refroidie ;
iv) ajout sous agitation à l'agitateur d'une quantité pharmaceutiquement acceptable de micafungine à la solution obtenue à l'étape (i), (ii) ou (iii) ;
v) ajustement du pH de la solution obtenue à l'étape (iv) à un pH entre 3,5 et 7,0, de préférence entre 4,6 et 6,0 en utilisant une quantité pharmaceutiquement acceptable d'un agent modifiant le pH ;
vi) facultativement, filtrage de la solution obtenue à l'étape (iv) ou (v) ;
vii) congélation de la solution obtenue à l'étape (iv), (v) ou (vi), de préférence cette étape est réalisée dans un flacon, plus préférablement, le flacon est scellé après lyophilisation sous atmosphère inerte, de préférence sous atmosphère d'azote ou d'argon ; et
viii) lyophilisation de la solution congelée obtenue à l'étape (vii), de préférence cette étape est réalisée dans un flacon, plus préférablement, le flacon est scellé après lyophilisation sous atmosphère inerte, de préférence sous atmosphère d'azote ou d'argon.

8. Procédé selon la revendication précédente dans lequel ladite étape de lyophilisation comprend les étapes de congélation de la solution, de séchage primaire et de séchage secondaire, et dans lequel la lyophilisation dure moins de 50 heures, de préférence entre 10 et 45 heures, et plus préférentiellement entre 25 et 40 heures depuis l'étape initiale de congélation de la solution jusqu'à la fin du séchage secondaire.

9. Procédé selon la revendication précédente, dans lequel la lyophilisation comprend les étapes suivantes :
i) congélation de la solution à une température inférieure à -20 °C et maintien de ladite température pendant au moins 1 heure, de préférence la température est entre -55 et -35 °C, plus préférentiellement entre -50 et -40 °C, maintenue au moins 2 heures, plus préférentiellement entre 2 et 4 heures ;
ii) séchage primaire de la solution congelée de l'étape (i) sous vide et à une température entre -10 et 10 °C, et maintien desdites conditions pendant au moins 10 heures, de préférence elle est entre -5 et 10 °C, maintenue au moins 12 heures, de préférence entre 15 et 25 heures ; et
iii) séchage secondaire de la solution lyophilisée primaire de l'étape (ii) sous vide et à une température entre 20 et 45 °C, de préférence la température est entre 25 et 40 °C, maintenue au moins 7 heures, plus préférentiellement entre 8 et 16 heures, et maintien desdites conditions pendant au moins 5 heures.

10. Procédé selon la revendication précédente, dans lequel le vide à l'étape (ii) est compris entre 1 Pa et 100 Pa (0.01 et 1 mbar), de préférence de 5 Pa à 50 Pa (0.05 à 0.5 mbar), plus préférablement de 10 Pa à 20 Pa (0.1 à 0.2 mbar) et/ou
le vide à l'étape (iii) est compris entre 0.5 Pa et 10 Pa (0.005 et 0.1 mbar), de préférence de 1 Pa à 8 Pa (0.01 à 0.08 mbar), plus préférablement de 2 Pa à 6 Pa (0.02 à 0.06 mbar).
